# EUROPEAN PATENT SPECIFICATION

(11) **EP 4 186 478 B1**
(45) Date of publication and mention of the grant of the patent: **28.01.2026**
(21) Application number: 22208609.2
(22) Date of filing: 21.11.2022
(51) Int. Cl.: A61F 2/966, A61M 25/00, A61F 2/07

(54) **RETRACTABLE, TAPERED TIP FOR CARDIOVASCULAR IMPLANT DELIVERY SYSTEMS**
EINZIEHBARE, KONISCHE SPITZE FÜR FREISETZUNGSSYSTEME FÜR HERZ-KREISLAUF-IMPLANTATE
POINTE EFFILÉE RÉTRACTABLE POUR SYSTÈMES DE POSE D'IMPLANT CARDIOVASCULAIRE

(30) Priority: 29.11.2021 US 202117536920
(43) Date of publication of application: 31.05.2023
(73) Proprietor: Medtronic Vascular Inc., Santa Rosa, CA 95403 (US)
(72) Inventor: Baranowski, Ian Benjamin, Santa Rosa, 95403 (US)
(74) Representative: Zimmermann & Partner Patentanwälte mbB

(56) References cited:
- US-A1- 2008 255 652
- US-A1- 2011 137 403
- US-A1- 2012 277 845
- US-A1- 2019 015 630

## Description

### TECHNICAL FIELD

The present disclosure relates generally to a retractable, tapered tip for implant prosthetic delivery systems.

### BACKGROUND

An endovascular procedure may be used to deliver a cardiovascular implant in a minimally invasive manner to a target cardiovascular site of a patient. One such endovascular procedure may utilize a stent graft as the cardiovascular implant. The stent graft has a surgical graft covering and an expanding (e.g., self-expanding) metal frame. The stent graft may be placed at a target cardiovascular site to bridge a damaged and/or diseased blood vessel and/or heart tissue at the target cardiovascular site, thereby restoring the function of the damaged and/or diseased blood vessel and/or heart tissue.

A target cardiovascular site may be accessed at a convenient and less traumatic entry point of the patient's body using an endovascular procedure. For instance, an implant (e.g., a stent graft) may be routed through the vasculature of a patient to the target cardiovascular site for deployment of the implant. Intraluminal deployment typically uses a delivery catheter with tubes or shafts arranged for relative axial movement. For example, an expandable stent graft may be compressed and disposed within a distal end of an outer shaft of the delivery catheter fixed to an inner shaft. The delivery catheter may then be maneuvered, typically tracked through a body lumen until a distal end of the delivery catheter and the stent graft are positioned at the target cardiovascular site. The expandable stent graft can then be deployed and radially expanded at the target cardiovascular site of the patient.

US 2012/277845 A1 describes a delivery system with a retractable proximal end.

US 2008/255652 A1 describes an expandable tip delivery system and method.

US 2011/137403 A1 describes an introducer for endovascular implants.

US 2019/015630 A1 describes a system and method for low-profile occlusion balloon catheter.

### SUMMARY

The invention is defined in claim 1. Further aspects and preferred embodiments are defined in the appended claims.

According to one embodiment, a cardiovascular implant delivery system is disclosed. The cardiovascular implant delivery system includes a retractable, tapered tip including a tapered portion tapering inward from a wide, proximal profile to a narrow, distal profile. The tapered portion has a non-retracted state and a retracted state. The delivery system also includes a cardiovascular implant implantable at a target cardiovascular site. The delivery system further includes an actuator configured to retract the tapered portion from the non-retracted state to the retracted state during advancement of the retractable, tapered tip and the cardiovascular implant toward the target cardiovascular site to reduce an axial length of the tapered portion.

According to another embodiment, a retractable, tapered tip for a cardiovascular implant delivery system is disclosed. The retractable, tapered tip includes a tapered portion tapering inward from a wide, proximal profile to a narrow, distal profile. The tapered portion has a non-retracted state and a retracted state. The tapered portion is configured to retract from the non-retracted state to the retracted state during advancement of the retractable, tapered tip and a cardiovascular implant toward a target cardiovascular site to reduce an axial length of the tapered portion.

According to an example, a method (not claimed) for delivering a cardiovascular implant to a target cardiovascular site. The method includes advancing a delivery system including a retractable, tapered tip and the cardiovascular implant toward the target cardiovascular site. The retractable, tapered tip has a non-retracted state and a retracted state. The method further includes retracting the retractable, tapered tip from the non-retracted state to the retracted state.

### BRIEF DESCRIPTION OF THE DRAWINGS

Figure 1A depicts an attempted delivery of a cardiovascular implant to a first target cardiovascular site using a non-retractable delivery tip of a cardiovascular implant delivery system.
Figure 1B depicts an attempted delivery of a cardiovascular implant to a second target cardiovascular site within a heart wall using a non-retractable delivery tip of a cardiovascular implant delivery system.
Figures 2A and 2B depict partial cross-sectional views of a retractable delivery tip of a cardiovascular delivery system configured to deliver a cardiovascular implant to a target cardiovascular site shown in cross section where the retractable delivery tip is shown in an undeformed state and a deformed state, respectively, according to one embodiment.
Figures 3A and 3B depict plan views of a retractable delivery tip of a cardiovascular delivery system configured to deliver a cardiovascular implant to a target cardiovascular site shown in cross section where the retractable delivery tip is shown in an undeformed state and a deformed state, respectively, according to one embodiment.
Figure 4A depicts a plan view of a delivery system including a tip deformation actuator configured to deform at least a portion of a delivery tip according to one embodiment.
Figure 4B depicts a magnified, plan view of the tip deformation actuator shown in Figure 4A.
Figure 5A depicts a plan view of a retractable delivery tip of a cardiovascular delivery system configured to deliver a cardiovascular implant to a target cardiovascular site where the retractable delivery tip is shown in an unbent state according to one embodiment.
Figure 5B depicts a plan view of the retractable delivery tip of Figure 5A where the retractable delivery tip has transitioned from the unbent state into a bent state according to one embodiment.
Figure 6A depicts a plan view of a retractable delivery tip of a cardiovascular delivery system configured to deliver a cardiovascular implant to a target cardiovascular site where the retractable delivery tip is shown in an unnested state according to one embodiment.
Figure 6B depicts a plan view of the retractable delivery tip of Figure 6A where the retractable delivery tip has transitioned from the unnested state to a nested state according to one embodiment.

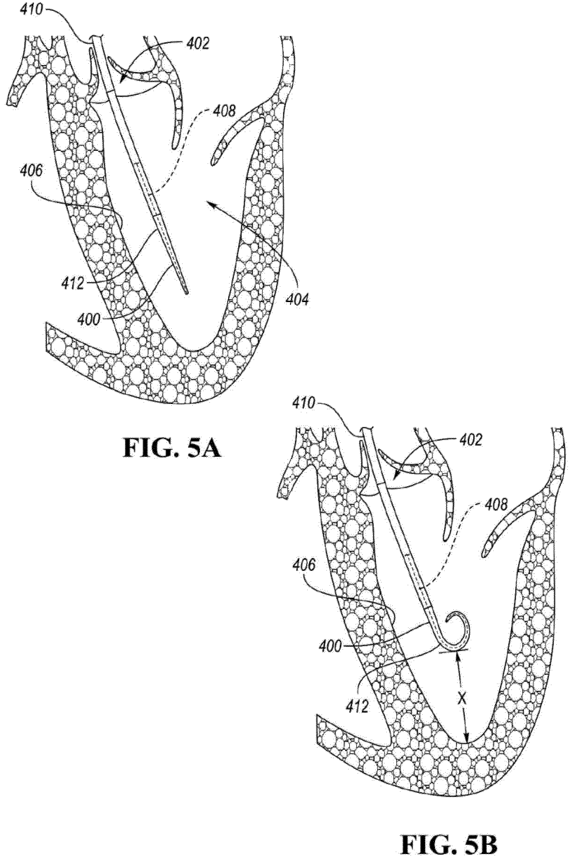

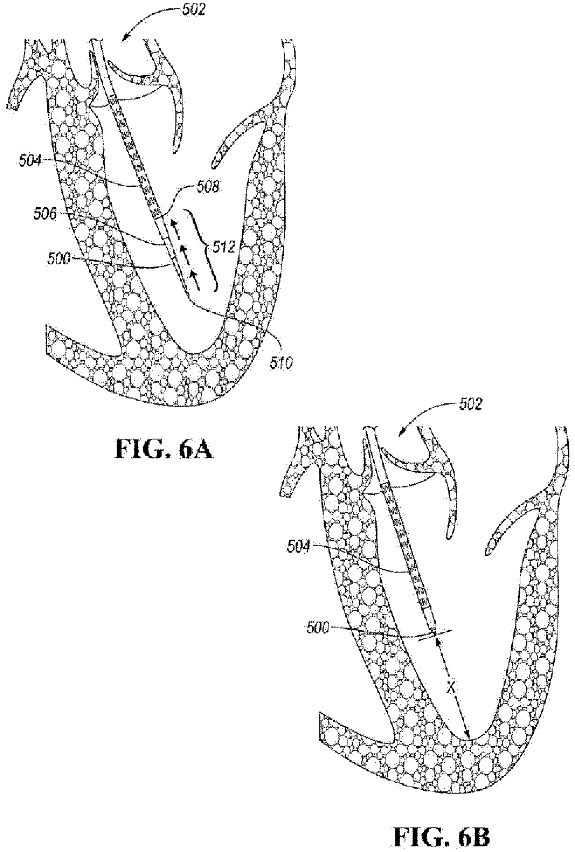

Configurations illustrated in fig. 5, 6 are not covered by the wording of the claims.

### DETAILED DESCRIPTION

Embodiments of the present disclosure are described herein. It is to be understood, however, that the disclosed embodiments are merely examples and other embodiments can take various and alternative forms. The figures are not necessarily to scale; some features could be exaggerated or minimized to show details of particular components. Therefore, specific structural and functional details disclosed herein are not to be interpreted as limiting, but merely as a representative basis for teaching one skilled in the art to variously employ the embodiments. As those of ordinary skill in the art will understand, various features illustrated and described with reference to any one of the figures can be combined with features illustrated in one or more other figures to produce embodiments that are not explicitly illustrated or described. The combinations of features illustrated provide representative embodiments for typical applications. Various combinations and modifications of the features consistent with the teachings of this disclosure, however, could be desired for particular applications or implementations.

Directional terms used herein are made with reference to the views and orientations shown in the exemplary figures. A central axis is shown in the figures and described below. Terms such as "outer" and "inner" are relative to the central axis. For example, an "outer" surface means that the surfaces faces away from the central axis, or is outboard of another "inner" surface. Terms such as "radial," "diameter," "circumference," etc. also are relative to the central axis. The terms "front," "rear," "upper" and "lower" designate directions in the drawings to which reference is made.

Unless otherwise indicated, for the delivery system the terms "distal" and "proximal" are used in the following description with respect to a position or direction relative to a treating clinician. "Distal" and "distally" are positions distant from or in a direction away from the clinician, and "proximal" and "proximally" are positions near or in a direction toward the clinician. For the stent-graft prosthesis, "proximal" is the portion nearer the heart by way of blood flow path while "distal" is the portion of the stent-graft further from the heart by way of blood flow path.

The following detailed description is merely exemplary in nature and is not intended to limit the invention or the application and uses of the invention. Although the description is in the context of treatment of heart tissue such as aortic valves, heart ventricles, and heart walls, and blood vessels such as the aorta, coronary, carotid, and renal arteries, the invention may also be used in any other body passageways where it is deemed useful.

Cardiovascular implants are devices configured to replace malfunctioning, nonfunctioning, damaged and/or diseased blood vessels and/or heart tissue. These devices may imitate the function, form, and/or shape of working blood vessels and/or heart tissue. Cardiovascular implants may include cardiovascular prosthetics.

Cardiovascular implants are delivered in a minimally invasive manner to avoid damage to vascular tissue and disruption of the function of surrounding anatomy during deployment. Cardiovascular implant delivery systems have been developed to address these concerns using a tapered tip at the distal end of the delivery system so that the delivery system may advance through the vasculature by the tapered tip gliding across the vessel tissue without dislodging material or puncturing tissue. The tapered tip includes a narrow, distal end and a broad, proximal end with a tapered body extending therebetween. In one configuration, the tapered tip has a frustoconical shape where the diameter increases from the narrow, distal end to the wide, proximal end. The diameter of the wide, proximal end may correspond to a diameter of an implant cover or sheath (e.g., a stent graft or prosthetic heart valve cover or sheath), which covers or sheaths an implant configured to be deployed at a target cardiovascular site.

The axial length of the tip is extended by tapering the tip. The tapered tip design spans a longer length in an axial direction than the tip would otherwise span because of the taper. Therefore, a tapered tip occupies a length distal the implant. The length distal the implant may preclude or hinder deployment of an implant in regions of the cardiovascular system that have a limited amount of axial travel due to anatomical constraints. Such regions may include an aortic valve and/or a heart wall.

Unless an access point different than the femoral access point is used, or the tapered tip is somehow removed after completing its tracking function, the implant may not accommodate patients that have short anatomical constraints. This problem may lead to fewer patients being treated by clinicians or clinicians pushing the limits of an implant delivery system, thereby potentially applying excessive pressure to delicate tissue within the cardiovascular system of a patient.

A tapered tip may provide one or more functions through its design. First, the tapered tip may track smoothly and dilate (e.g., enlarge and widen) anatomy while advancing to a target cardiovascular site. Second, the tapered tip may be designed to not interfere with an adjustment of a clinician to the deployment location. Third, the tapered tip may be designed to not interfere with cardiovascular tissue during deployment. Fourth, the tapered tip may be designed so as not to interfere with a previously implanted vascular device. And finally, the tapered tip may be designed to be removed smoothly and gently through the newly implanted device and exit anatomy.

According to one or more embodiments disclosed herein, a retractable, tapered tip for a cardiovascular delivery system is provided that provides one or more of the functions identified above while addressing delivery of a cardiovascular implant in a cardiovascular region having short anatomical constraints (e.g., an aortic valve and/or a heart wall). In one or more embodiments, a tapered tip that changes (e.g., reduces its axial length) in response to input from a clinician, such that a final deployment location (e.g., a target cardiovascular site) is achieved without interfering with anatomy in a cardiovascular region having short anatomical constraints (e.g., an aortic valve and/or a heart wall).

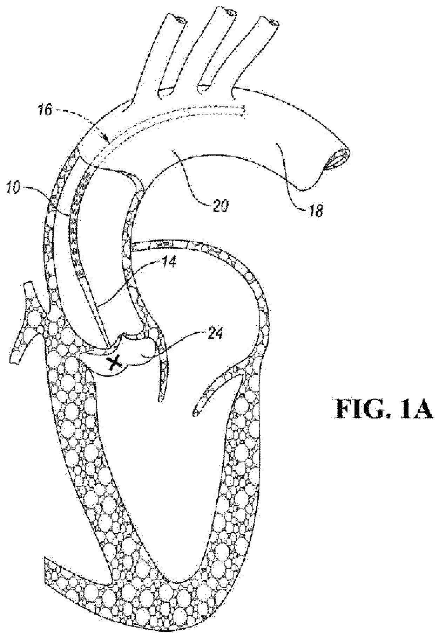

Figure 1A depicts an attempted delivery of cardiovascular implant 10 to a first target cardiovascular site using non-retractable delivery tip 14 of cardiovascular implant delivery system 16. The location of the first target cardiovascular site is merely an example, and it is to be understood that the location may vary from procedure to procedure. As shown in Figure 1A, non-retractable delivery tip 14 tracks cardiovascular implant delivery system 16 through aortic arch 18 and ascending aorta 20. Cardiovascular implant 10 includes cardiovascular implant length that matches the length of first target cardiovascular site. First target cardiovascular site is adjacent to aortic valve 24 and within ascending aorta 20. As represented by the X in Figure 1A, there is an interference between non-retractable delivery tip 14 and aortic valve 24 such that the cardiovascular implant length cannot match the position of the length of the first target cardiovascular site. Non-retractable delivery tip 14 interferes with the delivery of cardiovascular implant 10 to the first target cardiovascular site. In one or more embodiments, a retractable, tapered tip for cardiovascular implant delivery systems reduces or eliminates this interference such that a cardiovascular implant may be delivered to a first target cardiovascular site adjacent to an aortic valve and within the ascending aorta.

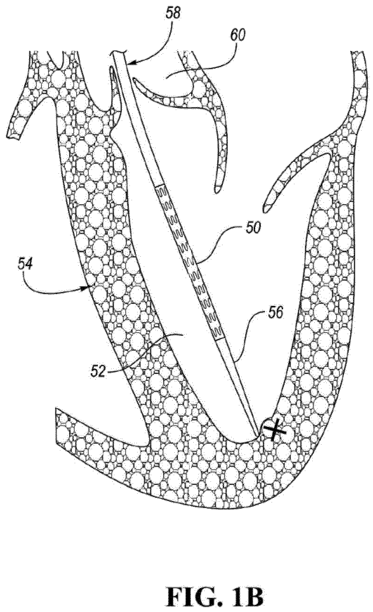

Figure 1B depicts an attempted delivery of cardiovascular implant 50 to second target cardiovascular site 52 within heart wall 54 using non-retractable delivery tip 56 of cardiovascular implant delivery system 58. The location of second target cardiovascular site 52 is merely an example, and it is to be understood that the location may vary from procedure to procedure. As shown in Figure 1B, non-retractable delivery tip 56 tracks cardiovascular implant delivery system 58 through aortic valve 60 into a region within heart wall 54. As represented by the X in Figure 1B, there is an interference between non-retractable delivery tip 56 and a distal region within heart wall 54 such that cardiovascular implant 50 does not reach second target cardiovascular site 52. Non-retractable delivery tip 56 interferes with the delivery of cardiovascular implant 50 to second target cardiovascular site 52. In one or more embodiments, a retractable, tapered tip for cardiovascular implant delivery systems reduces or eliminates this interference such that a cardiovascular implant may be delivered to a second target cardiovascular site in a region within the heart wall.

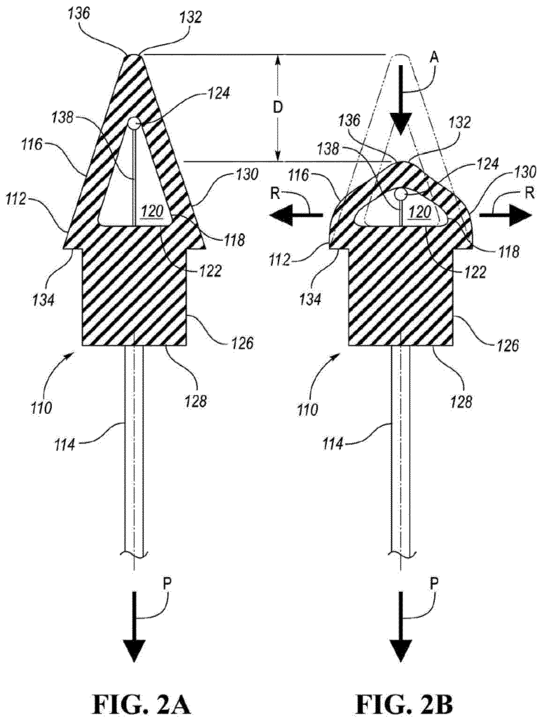

Figures 2A and 2B depict delivery tip system 110 of a delivery system configured to deliver a cardiovascular implant to a target cardiovascular site where delivery tip system 110 is shown in an undeformed state and a deformed state, respectively, according to one embodiment. Delivery tip system 110 includes delivery tip 112 and lumen 114, which is operatively connected to delivery tip 112. Delivery tip 112 has outer surface 116 and inner surface 118 with a thickness extending therebetween. Inner surface 118 defines inner cavity 120. Inner surface 118 may surround inner cavity 120 such that inner cavity 120 does not communicate with blood within the cardiovascular anatomy during deployment of delivery tip system 110. Inner surface 118 tapers inward from proximal end 122 to distal end 124. Delivery tip 112 includes base portion 126 adjacent to proximal end 128 of delivery tip 112 and tapered portion 130 adjacent to distal end 132 of delivery tip 112. Tapered portion 130 tapers inward from proximal end 134 to distal end 136. Tapered portion 130 is tapered so that delivery tip system 110 moves smoothly across vascular tissue without damaging (e.g., puncturing or perforating) tissue and/or dislodge material (e.g., plaque) as delivery tip system 110 is advanced through a patient's vasculature. As shown in Figures 2A and 2B, the profiles of inner cavity 120 and tapered portion 130 may be similar with the profile of inner cavity 120 being smaller in size than the profile of tapered portion 130. In one or more other embodiments, the profile of inner cavity 120 may be different than the profile of tapered portion 130.

At least a portion of delivery tip 112 is formed of a deformable material (e.g., a material configured to change in size or shape in response to an application of a force). In one or more embodiments, tapered portion 130 of delivery tip 112 is formed of a deformable material while base portion 126 is formed of a rigid or semi-rigid material so that delivery tip 112 deforms against base portion 126. Base portion 126 is solid and does not include an inner cavity to enhance the deformability of tapered portion 130 against base portion 126. In this embodiment, tapered portion 130 and base portion 126 may be co-molded to each other. Non-limiting examples of deformable materials include silicone, deformable polymer materials such as urethane-coated silicone, polycarbonate-urethane, and urethane-coated silicone, and deformable metal materials such as NiTi alloy and stainless steel. At least a portion of delivery tip 112 (e.g., tapered portion 130) may be formed of a deformable metal material embedded in a deformable polymer material depending on the strength and deformation characteristics identified for one or more delivery applications.

Wire 138 is secured to a portion of distal end 124 of inner surface 118 through a fastener such as a suture, stitch, anchor, or hook, or a distal end of the sire 138 may have an anchor portion embedded in the wall of the tapered portion 130. In one or more embodiments, wire 138 extends from distal end 124 of inner surface 118 through inner cavity 120 and lumen 114 to a handle portion of the delivery system. Wire 138 is operatively connected to the handle portion of the delivery system such that the handle may be actuated to exert pulling force P on wire 138 in a direction toward the clinician (i.e., a force in the proximal direction and away from the distal direction of the delivery system). The pulling force P deforms the deformable material of delivery tip 112 (e.g., tapered portion 130) such that the deformable material deforms in an outward radial direction R and an axial direction A. Radial direction R is generally orthogonal to axial direction A. The deformable material of delivery tip 112 may deform into inner cavity 120 such that inner cavity 120 acts as a crumple zone to control the deformation of delivery tip 112.

The deformation of the deformable material of delivery tip 112 decreases the axial length of delivery tip 112 such that a target cardiovascular site is achieved without interfering with the anatomy in a cardiovascular region having short anatomical constraints. The axial length decrease may be any of the following values or in a range of any two of the following values: 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, and 3.0 centimeters.

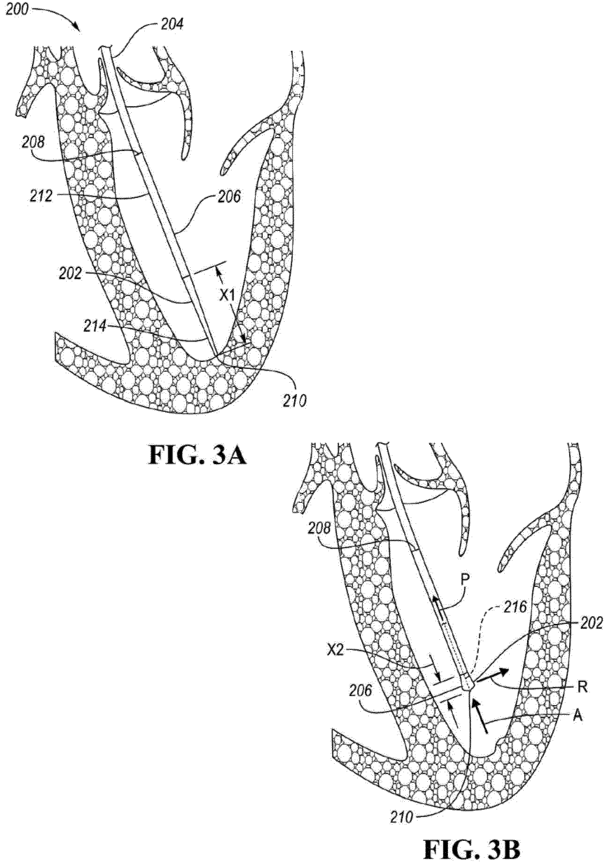

Figures 3A and 3B depict delivery tip system 200 of a delivery system configured to deliver a cardiovascular implant to a target cardiovascular site where delivery tip system 200 is shown in an undeformed state and a deformed state, respectively, according to one embodiment. Delivery tip system 200 includes delivery tip 202 and lumen 204, which is operatively connected to delivery tip 202. Delivery tip 202 has an outer surface and an inner surface with a thickness extending therebetween. The inner surface defines inner cavity 206. The inner surface may surround inner cavity 206 such that inner cavity 206 does not communicate with blood within the cardiovascular anatomy during deployment of delivery tip system 200. The inner surface tapers inward from proximal end 208 to distal end 210. Delivery tip 202 includes base portion 212 adjacent to proximal end 208 of delivery tip 202 and tapered portion 214 adjacent to distal end 210 of delivery tip 202. Base portion 212 and tapered portion 214 may collectively form an integral delivery tip 202. Tapered portion 214 tapers inward from proximal end 208 to distal end 210. Tapered portion 214 is tapered so that delivery tip system 200 moves smoothly across vascular tissue without damaging (e.g., puncturing or perforating) tissue and/or dislodge material (e.g., plaque) as delivery tip system 200 is advanced through a patient's vasculature. As shown in Figures 3A and 3B, the profiles of inner cavity 206 and delivery tip 202 may be similar with the profile of inner cavity 206 being smaller in size than the profile of delivery tip 202. In one or more other embodiments, the profile of inner cavity 206 may be different than the profile of delivery tip 202.

In an example (not claimed), at least a portion of base portion 212 may be formed of a deformable material. In one or more embodiments, tapered portion 214 of delivery tip 202 may be formed of a deformable material while base portion 212 is formed of a rigid or semi-rigid material so that delivery tip 202 deforms against base portion 212. In this embodiment, tapered portion 214 and base portion 212 may be co-molded to each other. Non-limiting examples of deformable materials include deformable polymer materials such as those disclosed above, and deformable metal materials such as those disclosed above. At least a portion of delivery tip 202 (e.g., tapered portion 214) may be formed of a deformable metal material embedded in a deformable polymer material depending on the strength and deformation characteristics identified for one or more delivery applications. In another embodiment, the entire delivery tip 212 is formed of a deformable material. The delivery tip 212 may have a uniform thickness surrounding inner cavity 206.

Wire 216 is secured to a portion of distal end 210 through a fastener such as a suture, stitch, anchor, or hook, or a distal end of the wire 138 may have an anchor portion embedded in the wall of the tapered portion 130. In one or more embodiments, wire 216 extends from distal end 210 through inner cavity 206 and lumen 204 to a handle portion of the delivery system. Wire 216 is connected to the handle portion of the delivery system such that the handle portion may be actuated to exert pulling force P on wire 216 in a direction toward the clinician (i.e., a force in the proximal direction and away from the distal direction of the delivery system). The pulling force P deforms the deformable material of delivery tip 202 (e.g., tapered portion 214) such that the deformable material deforms in an outward radial direction R and an axial direction A. Radial direction R is generally orthogonal to axial direction A. The deformable material of delivery tip 202 may deform into inner cavity 206.

Axial direction A and pulling force P are the same direction in one or more embodiments. The deformation of the deformable material of delivery tip 202 decreases the axial length of delivery tip 202 such that a target cardiovascular site is achieved without interfering with the anatomy in a cardiovascular region having short anatomical constraints. The axial length decrease from X1 to X2 may be any of the following values or in a range of any two of the following values: 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, and 3.0 centimeters.

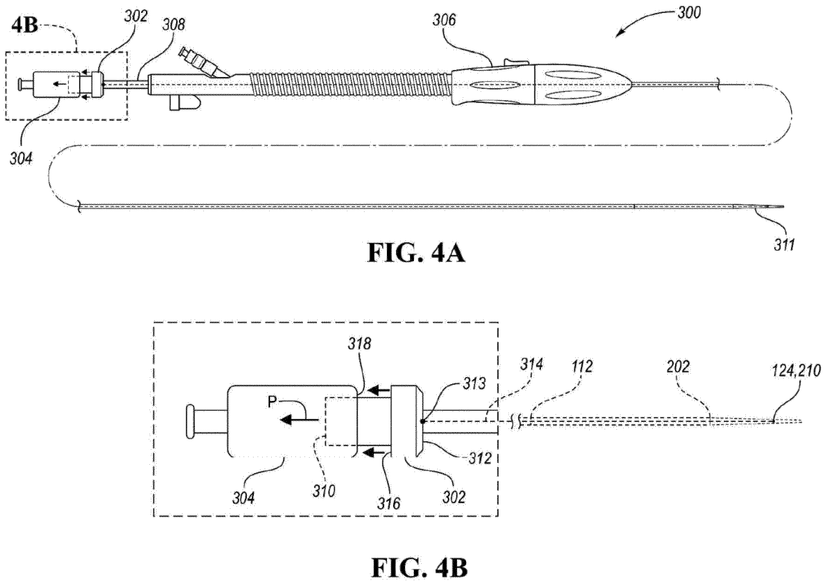

Figure 4A depicts delivery system 300 including tip deformation actuator 302 configured to deform at least a portion of delivery tip 112 or 202 according to one embodiment. Figure 4B depicts a magnified, plan view of tip deformation actuator 302 shown in Figure 4A. Delivery system 300 also includes tip release actuator 304, as shown in Figures 4A and 4B. Tip release actuator 304 is operatively connected to a tip capture mechanism, in which manipulation (e.g., rotation) of tip release actuator 304 can release a tip capture mechanism from a cardiovascular implant (e.g., a stent graft) (not shown) to deploy the cardiovascular implant at a target cardiovascular site. Delivery system 300 may also include handle 306 operatively connected to a stent graft cover retraction mechanism, in which manipulation (e.g., rotation) of the handle relative to a threaded shaft can retract the stent graft cover, allowing the stent graft to expand radially outward.

Delivery system 300 includes lumen 308 that is hollow. Lumen 308 extends from tip release actuator 304 through tip deformation actuator 302 and handle 306. Lumen 308 extends further to delivery tip 311. Tip deformation actuator 302 has proximal end 310 and distal end 312. Wire 314 is attached to distal end 312 via fastener 313. Wire 314 extends through lumen 308 to distal end 124 of inner surface 118 of delivery tip 112 or distal end 210 if delivery tip 202. During deployment of a cardiovascular implant, tip deformation actuator 302 may be manipulated to move tip deformation actuator 302 in a proximal direction P, thereby deforming the deformable material of delivery tip 112 (e.g., delivery portion 130) or delivery tip 202 (e.g., tapered portion 214) to reduce the axial length of delivery tip 112 or 202. In one embodiment, tip deformation actuator 302 may be in threaded engagement with tip release actuator 304 such that rotational movement of tip deformation actuator 302 into tip release actuator 304 moves tip deformation actuator 302 into tip release actuator 304 and holds wire 314 in a proximal position to maintain the deformation of delivery tip 112 or 202 while the cardiovascular implant is being deployed. As shown in Figure 4B, tip deformation actuator 302 includes intermediate surface 316 and tip release actuator 304 includes distal end 318. Tip deformation actuator 302 may be manipulated in a proximal direction of the delivery system from a distal position (shown in Figure 4B) to a proximal position where movement of tip deformation actuator 302 stops when intermediate surface 316 stops on distal end 318 of tip release actuator 304. The distance between the distal position and the proximal position may match the axial reduction in distance of the retractable tip through deformation (e.g., distance D of Figures 2A and 2B or the difference between distances X1 and X2 in Figures 3A and 3B).

Figure 5A depicts a plan view of retractable delivery tip 400 of cardiovascular delivery system 402 configured to deliver cardiovascular implant to target cardiovascular site 404 within heart wall 406 where retractable delivery tip 400 is shown in an unbent state according to one embodiment. Figure 5B depicts side view of retractable delivery tip 400 where retractable delivery tip 400 has transitioned from the unbent state into a bent state.

The retractable delivery tip 400 may be formed of a deformable material. A distal portion of retractable delivery tip 400 includes spine 408 formed of a shape memory material or a hyperelastic material. The shape memory material may be a material that exists in a pre-deformed state (e.g., a pre-deformed shape) and a deformed state (e.g., a deformed shape). The shape memory material may be configured to deform from its pre-deformed state into its deformed state under a first condition (e.g., cold deformation) and to return to its pre-deformed state when a second condition (e.g., a stimulus) is applied. The first condition may be ambient conditions. The second condition may be a stimulus applied by heat, electricity, or mechanical actuation. The heat stimulus may heat the shape memory material to a transition temperature where the shape memory material transitions from its deformed state to its pre-deformed state. The electrical stimulus may be the application of a shielded or insulated current to the shape memory material so that the shape memory material transitions from its deformed state to its pre-deformed state. Non-limiting examples of shape memory materials include shape memory alloys and shape memory polymers. Non-limiting examples of shape memory alloys for application in the retractable, tapered tips of one or more embodiments include NiTi alloys, such as Nitinol, and Co alloys. Non-limiting examples of shape memory polymers for application in the retractable, tapered tips of one or more embodiments include thermoplastic elastomers, multiblock copolymers of macrodiols based on pentadeclaracton (PDL) and caprolacton (PCL) and a diisocyanate, polymer networks, amorphous polyurethane networks of trioles and/or tetroles and diisocyanate, and functionalized macrodiols. A hyperelastic material may be configured to reversibly deform to a high strain from a pre-deformed state to a deformed state in response to a high stress.

Retractable delivery tip 400 includes base portion 410 at a proximal region of retractable delivery tip 400 and tapered portion 412 at a distal region of retractable delivery tip 400. As shown in Figures 5A and 5B, spine 408 extends the entire length of tapered portion 412. The percentage extension of the length of spine 408 in tapered portion 412 may be any of the following percentages or in a range of any two of the following percentages: 50%, 55%, 60%, 65%, 70%, 75%, 80%, 85%, 90%, 95%, and 100%. Spine 408 may be partially or entirely embedded within the thickness of retractable delivery tip 400. Spine 408 may be secured to the inner or outer surface of tapered portion 412. As shown in Figures 5A and 5B, spine 408 has a linear deformed shape and a curled pre-deformed shape, respectively. The curled pre-deformed shape reduces the axial length of retractable delivery tip 400 by the dimension X, as shown in Figure 5B. The curled pre-deformed shape shown in Figure 5B curls inward in a plane including retractable delivery tip 400. In another embodiment, the pre-deformed shape may be a helical shape wrapped around the longitudinal axis of retractable delivery tip 400. The deformed shape may extend beyond the axial cross-sectional profile of base portion 410 of retractable delivery tip 400.

In another embodiment, tapered portion 412 itself may be formed from a shape memory material or a hyperelastic material and may be configured to transition from a linear/straight configuration as shown in Figure 5A to a curled or helical configuration as shown in Figure 5B. In this embodiment, spine 408 may be configured to maintain tapered portion 412 in the linear configuration (e.g., deformed state) and upon its removal from tapered portion 412, the latter may transition to the curled configuration (e.g., pre-deformed or natural state). Spine 408 may be attached to an actuator, similar to in delivery system 300, or it may extend all the way back to and out of the handle such that it can be manually retracted by the operator.

Figures 6A and 6B depict plan views of retractable delivery tip 500 of cardiovascular delivery system 502 configured to deliver cardiovascular implant 504. Figure 6A shows retractable delivery tip 500 in an unnested state. Figure 6B shows retractable delivery tip 500 in a nested state.

Retractable delivery tip 500 includes tapered portion 506 decreasing in diameter from wide proximal portion 508 to narrow distal portion 510. Tapered portion 506 includes a number of links 512 or sections/portions. As shown in Figure 6A, each of the number of links 512 may be tapered and collectively form tapered portion 506. Each successive link 512 from wide proximal portion 508 to narrow distal portion 510 may reduce in diameter along the length of each successive link to form an overall tapered shape of tapered portion 506. Each adjacent pair of the number of links 512 is connected to each other such that the number of links 512 do not separate from each other during deployment or retrieval of cardiovascular implant 504. As shown in Figure 6A, each adjacent pair of the number of links 512 is interlocked such that tapered portion 506 does not collapse while tracking to a target cardiovascular site. Prior to advancing to the target cardiovascular site, an actuator may be configured to unlock adjacent pairs of the number of links 512 such that the number of links 512 collapse into each other to form a nested state of retractable delivery tip 500 as shown in Figure 6B. The actuator may be located near the proximal end of the delivery system and connected to a wire that is also connected to retractable delivery tip 500. Actuation of the wire by the actuator makes the number of links 512 collapse on each other as shown in Figure 6B. The actuation may cause relative rotational movement between the links such that adjacent links become unlocked from each other, and thereby collapse. The collapsing of retractable delivery tip 500 into the nested state reduces the axial length of retractable delivery tip 500 by the dimension X, as shown in Figure 6B. The collapsing of retractable delivery tip 500 into the nested state reduces the axial length thereby allowing relatively shorter landing zones where anatomical structures may otherwise interfere with the delivery tip system, such as in the ascending aortic region and the heart valve region.

While exemplary embodiments are described above, it is not intended that these embodiments describe all possible forms encompassed by the claims. The words used in the specification are words of description rather than limitation, and it is understood that various changes can be made without departing from the scope of the disclosure. As previously described, the features of various embodiments can be combined to form further embodiments of the invention that may not be explicitly described or illustrated. While various embodiments could have been described as providing advantages or being preferred over other embodiments or prior art implementations with respect to one or more desired characteristics, those of ordinary skill in the art recognize that one or more features or characteristics can be compromised to achieve desired overall system attributes, which depend on the specific application and implementation. These attributes can include, but are not limited to cost, strength, durability, life cycle cost, marketability, appearance, packaging, size, serviceability, weight, manufacturability, ease ofassembly, etc. As such, to the extent any embodiments are described as less desirable than other embodiments or prior art implementations with respect to one or more characteristics, these embodiments are not outside the scope of the disclosure and can be desirable for particular applications.

## Claims

1. A retractable, tapered tip for a cardiovascular implant delivery system (10), the retractable, tapered tip comprising:
a retractable, tapered tip (112) including a tapered portion (130) tapering inward from a wide, proximal profile to a narrow, distal profile, the tapered portion having a non-retracted state and a retracted state, the tapered portion configured to retract from the non-retracted state to the retracted state during advancement of the retractable, tapered tip and a cardiovascular implant toward a target cardiovascular site to reduce an axial length of the tapered portion;
wherein the retractable, tapered tip includes a base portion (126), the tapered portion extends from the base portion;
wherein the tapered portion (130) is formed of a deformable material and the base portion is formed of a rigid or semi-rigid material so that the tapered portion (130) deforms against the base portion (126), and wherein the base portion (126) is solid and does not include an inner cavity to enhance the deformability of the tapered portion (130) against the base portion (126),
wherein the tapered tip includes outer and inner surfaces (116, 118), the inner surface (118) of the tapered tip defines a cavity (120) within the tapered tip, and
wherein the base portion has a constant profile along its length, and the base portion and the tapered portion collectively define the cavity (120) within the retractable, tapered tip.

2. The retractable, tapered tip of claim 1, wherein the deformable material is a deformable polymer, a deformable metal, or a combination thereof.

3. A cardiovascular implant delivery system (110) comprising:
the retractable, tapered tip (112) of claim 1;
a cardiovascular implant implantable at a target cardiovascular site; and
an actuator (302) configured to retract the tapered portion from the non-retracted state to the retracted state during advancement of the retractable, tapered tip and the cardiovascular implant toward the target cardiovascular site to reduce an axial length of the tapered portion.

4. The cardiovascular implant delivery system of claim 3, wherein the actuator includes a handle portion disposed at a proximal region of the cardiovascular implant delivery system and a wire (138) operatively connected to the handle portion and the tapered tip, the handle portion is configured to generate a pulling force on the wire in a proximal direction of the cardiovascular implant delivery system to reduce the axial length of the tapered portion.

5. The cardiovascular implant delivery system of claim 4, wherein the handle portion is rotatable in the proximal direction of the cardiovascular implant delivery system to reduce the axial length of the tapered portion.

6. The cardiovascular implant delivery system of claim 4, wherein the wire is secured to the inner surface.

7. The cardiovascular implant delivery system of claim 4, wherein the handle portion is rotatable between an initial position where the tapered portion has the non-retracted state and a final position where the handle portion contacts a stop on a second handle portion.

8. The cardiovascular implant delivery system of claim 4. wherein the wire (314) is operatively connected to the handle portion via a fastener (313).

## Patentansprüche

1. Einziehbare, sich verjüngende Spitze für ein Abgabesystem (10) für ein kardiovaskuläres Implantat, wobei die einziehbare, sich verjüngende Spitze umfasst:
eine einziehbare, sich verjüngende Spitze (112), einschließlich eines sich verjüngenden Abschnitts (130), der sich von einem breiten, proximalen Profil zu einem schmalen, distalen Profil nach innen verjüngt, wobei der sich verjüngende Abschnitt einen nicht-eingezogenen Zustand und einen eingezogenen Zustand aufweist, wobei der sich verjüngende Abschnitt konfiguriert ist, um sich von dem nicht-eingezogenen Zustand in den eingezogenen Zustand während des Vorschiebens der einziehbaren, sich verjüngenden Spitze und eines kardiovaskulären Implantats zu einer kardiovaskulären Zielstelle hin einzuziehen, um eine axiale Länge des sich verjüngenden Abschnitts zu reduzieren;
wobei die einziehbare, sich verjüngende Spitze einen Basisabschnitt (126) einschließt, wobei sich der sich verjüngende Abschnitt von dem Basisabschnitt erstreckt;
wobei der sich verjüngende Abschnitt (130) aus einem verformbaren Material ausgebildet ist und der Basisabschnitt aus einem starren oder halbstarren Material ausgebildet ist, sodass sich der sich verjüngende Abschnitt (130) gegen den Basisabschnitt (126) verformt, und wobei der Basisabschnitt (126) massiv ist und keinen Innenhohlraum einschließt, um die Verformbarkeit des sich verjüngenden Abschnitts (130) gegen den Basisabschnitt (126) zu verbessern,
wobei die sich verjüngende Spitze eine äußere und eine innere Oberfläche (116, 118) einschließt, wobei die innere Oberfläche (118) der sich verjüngenden Spitze einen Hohlraum (120) innerhalb der sich verjüngenden Spitze definiert, und
wobei der Basisabschnitt ein konstantes Profil entlang seiner Länge aufweist, und der Basisabschnitt und der sich verjüngende Abschnitt gemeinsam den Hohlraum (120) innerhalb der einziehbaren, sich verjüngenden Spitze definieren.

2. Einziehbare, sich verjüngende Spitze nach Anspruch 1, wobei das verformbare Material ein verformbares Polymer, ein verformbares Metall oder eine Kombination davon ist.

3. Abgabesystem (110) für ein kardiovaskuläres Implantat, umfassend:
die einziehbare, sich verjüngende Spitze (112) von Anspruch 1;
ein kardiovaskuläres Implantat, das an einer kardiovaskulären Zielstelle implantierbar ist; und
einen Aktuator (302), der konfiguriert ist, um den sich verjüngenden Abschnitt aus dem nicht-eingezogenen Zustand in den eingezogenen Zustand während des Vorschiebens der einziehbaren, sich verjüngenden Spitze und des kardiovaskulären Implantats zu der kardiovaskulären Zielstelle hin einzuziehen, um eine axiale Länge des sich verjüngenden Abschnitts zu reduzieren.

4. Abgabesystem für ein kardiovaskuläres Implantat nach Anspruch 3, wobei der Aktuator einen Griffabschnitt, der in einem proximalen Bereich des Abgabesystems für ein kardiovaskuläres Implantat angeordnet ist, und einen Draht (138) einschließt, der mit dem Griffabschnitt und der sich verjüngenden Spitze wirkverbunden ist, wobei der Griffabschnitt konfiguriert ist, um eine Zugkraft auf den Draht in einer proximalen Richtung des Abgabesystems für ein kardiovaskuläres Implantat zu erzeugen, um die axiale Länge des konischen Abschnitts zu reduzieren.

5. Abgabesystem für ein kardiovaskuläres Implantat nach Anspruch 4, wobei der Griffabschnitt in der proximalen Richtung des Abgabesystems für ein kardiovaskuläres Implantat drehbar ist, um die axiale Länge des sich verjüngenden Abschnitts zu reduzieren.

6. Abgabesystem für ein kardiovaskuläres Implantat nach Anspruch 4, wobei der Draht an der inneren Oberfläche befestigt ist.

7. Abgabesystem für ein kardiovaskuläres Implantat nach Anspruch 4, wobei der Griffabschnitt zwischen einer Anfangsposition, in der der sich verjüngende Abschnitt den nicht-eingezogenen Zustand aufweist, und einer Endposition, in der der Griffabschnitt einen Anschlag an einem zweiten Griffabschnitt kontaktiert, drehbar ist.

8. Abgabesystem für ein kardiovaskuläres Implantat nach Anspruch 4, wobei der Draht (314) mit dem Griffabschnitt über ein Befestigungsmittel (313) wirkverbunden ist.

## Revendications

1. Pointe rétractable et effilée pour un système de pose d'implant cardiovasculaire (10), la pointe rétractable et effilée comprenant :
une pointe rétractable et effilée (112) comportant une partie effilée (130) s'effilant vers l'intérieur à partir d'un profil large et proximal vers un profil étroit et distal, la partie effilée ayant un état non rétracté et un état rétracté, la partie effilée conçue pour se rétracter de l'état non rétracté à l'état rétracté pendant l'avancement de la pointe rétractable et effilée et d'un implant cardiovasculaire vers un site cardiovasculaire cible afin de réduire une longueur axiale de la partie effilée ;
dans laquelle la pointe rétractable et effilée comporte une partie de base (126), la partie effilée s'étend à partir de la partie de base ;
dans laquelle la partie effilée (130) est formée d'un matériau déformable et la partie de base est formée d'un matériau rigide ou semi-rigide de sorte que la partie effilée (130) se déforme contre la partie de base (126), et dans laquelle la partie de base (126) est solide et ne comporte pas de cavité intérieure pour améliorer la déformabilité de la partie effilée (130) contre la partie de base (126),
dans laquelle la pointe effilée comporte des surfaces extérieures et intérieures (116, 118), la surface intérieure (118) de la pointe effilée définit une cavité (120) à l'intérieur de la pointe effilée, et
dans laquelle la partie de base a un profil constant sur toute sa longueur, et la partie de base et la partie effilée définissent collectivement la cavité (120) à l'intérieur de la pointe rétractable et effilée.

2. Pointe rétractable et effilée selon la revendication 1, dans laquelle le matériau déformable est un polymère déformable, un métal déformable ou une combinaison de ceux-ci.

3. Système de pose d'implant cardiovasculaire (110), comprenant :
la pointe rétractable et effilée (112) selon la revendication 1 ;
un implant cardiovasculaire implantable sur un site cardiovasculaire cible ; et
un actionneur (302) conçu pour rétracter la partie effilée de l'état non rétracté à l'état rétracté pendant l'avancement de la pointe effilée rétractable et de l'implant cardiovasculaire vers le site cardiovasculaire cible afin de réduire la longueur axiale de la partie effilée.

4. Système de pose d'implant cardiovasculaire selon la revendication 3, dans lequel l'actionneur comporte une partie de poignée disposée dans une région proximale du système de pose d'implant cardiovasculaire et un fil (138) relié de manière opérationnelle à la partie de poignée et à la pointe effilée, la partie de poignée est conçue pour générer une force de traction sur le fil dans une direction proximale du système de pose d'implant cardiovasculaire afin de réduire la longueur axiale de la partie effilée.

5. Système de pose d'implant cardiovasculaire selon la revendication 4, dans lequel la partie poignée est rotative dans la direction proximale du système de pose d'implant cardiovasculaire afin de réduire la longueur axiale de la partie effilée.

6. Système de pose d'implant cardiovasculaire selon la revendication 4, dans lequel le fil est fixé à la surface intérieure.

7. Système de pose d'implant cardiovasculaire selon la revendication 4, dans lequel la partie poignée est rotative entre une position initiale dans laquelle la partie effilée est non rétractée et une position finale dans laquelle la partie poignée entre en contact avec une butée sur une seconde partie poignée.

8. Système de pose d'implant cardiovasculaire selon la revendication 4, dans lequel le fil (314) est relié de manière opérationnelle à la partie poignée par l'intermédiaire d'une attache (313).
